**Europäisches Patentamt**

**European Patent Office**

**Office européen des brevets**

(19)

(11) Veröffentlichungsnummer : **0 369 439 B1**

(12) # EUROPÄISCHE PATENTSCHRIFT

(45) Veröffentlichungstag der Patentschrift :
**24.03.93 Patentblatt 93/12**

(51) Int. Cl.⁵ : **B01J 12/00,** B01J 35/04, C07B 39/00, // C07C17/00, C07C17/10, C07C17/154, C07C17/156

(21) Anmeldenummer : **89121182.3**

(22) Anmeldetag : **16.11.89**

(54) **Verwendung von wabenförmigen monolithischen Katalysatorträgern für Chlorierungs- und Oxichlorierungsreaktionen.**

Die Akte enthält technische Angaben, die nach dem Eingang der Anmeldung eingereicht wurden und die nicht in dieser Patentschrift enthalten sind.

(30) Priorität : **17.11.88 DE 3838811**

(43) Veröffentlichungstag der Anmeldung :
**23.05.90 Patentblatt 90/21**

(45) Bekanntmachung des Hinweises auf die Patenterteilung :
**24.03.93 Patentblatt 93/12**

(84) Benannte Vertragsstaaten :
**BE DE FR GB IT NL SE**

(56) Entgegenhaltungen :
**DE-A- 3 213 413**
**DE-A- 3 334 223**
**GB-A- 980 983**

(73) Patentinhaber : **WACKER-CHEMIE GMBH**
**Hanns-Seidel-Platz 4**
**W-8000 München 83 (DE)**
Patentinhaber : **Degussa Aktiengesellschaft**
**Weissfrauenstrasse 9**
**W-6000 Frankfurt am Main 1 (DE)**

(72) Erfinder : **Strasser, Rudolf, Dr. Dipl.-Chem.**
**Lindacherstrasse 58**
**W-8263 Burghausen (DE)**
Erfinder : **Schmidhammer, Ludwig, Dr. Dipl.-Chem.**
**Pappelweg 5**
**W-8261 Haiming (DE)**
Erfinder : **Deller, Klaus, Dr. Dipl.-Ing.**
**Friedhofstrasse 47**
**W-6452 Hainburg (DE)**
Erfinder : **Krause, Helmfried**
**Odenwaldstrasse 39**
**W-6458 Rodenbach (DE)**

## Beschreibung

Die Erfindung betrifft die Verwendung von wabenförmigen monolithischen Katalysatorträgern vorzugsweise für selektive Chlorierungs- und/oder Oxichlorierungsreaktionen in Rohrbündelreaktoren mit Festbettanordnung des Katalysators.

Derartige Reaktionen dienen zur Herstellung von gesättigten oder ungesättigten Mono- bzw. Polychlorkohlenwasserstoffen aus gesättigten und ungesättigten Kohlenwasserstoffen bzw. partiell chlorierten Kohlenwasserstoffen.

Selektive Chlorierungs- und/oder Oxichlorierungsreaktionen, zum Beispiel die Umsetzung von ethylenhaltigen Abgasen mit Chlor zu 1.2-Dichlorethan, oder die Umsetzung von Ethylen mit Chlorwasserstoff und Luft und/oder Sauerstoff zu 1.2-Dichlorethan und Wasser oder die Umsetzung von Methan mit Chlor und/oder Chlorwasserstoff und Luft und/oder Sauerstoff zu chlorierten Methanen, oder die Umsetzung von Ethan mit Chlor zu Vinylchlorid und chlorierten Ethanen, werden häufig in Reaktoren an Festbettkatalysatoren durchgeführt, wobei die Reaktanden gasförmig vorliegen. Dabei spielt sich die eigentliche Reaktion im Übergangsbereich Gas-Festzustand ab. Die gasförmigen Reaktanden passieren den Katalysator, die feste Phase, gewöhnlich bei erhöhten Temperaturen und Drucken und reagieren an der Phasengrenze Gas-Festzustand miteinander. Die dabei entstehenden Produkte verlassen mit dem nicht umgesetzten Anteil des Gasstromes den Reaktor und werden anschließend durch geeignete verfahrenstechnische Maßnahmen von dem nicht umgesetzten Anteil angetrennt.

Die eben genannten Reaktionen sind überwiegend stark exotherm, weshalb zur besseren Wärmebeherrschung vielfach die Reaktion durch Inserieschaltung mehrerer Reaktoren quasi auseinandergezogen wird. Die Kühlung der Reaktoren erfolgt dabei üblicherweise durch Verdampfen von heißem Druckwasser oder mit Hilfe eines Wärmeträgermediums, das, je nach erforderlichem Temperaturniveau, zum Beispiel eine Kohlenwasserstoff-Fraktion, ein Thermoöl oder eine Salzschmelze sein kann. Konventionell bestehen derartige Reaktoren für exotherme Reaktionen aus vielen Rohren, in denen sich der Katalysator befindet und die von einem Kühlmantel umgeben sind, in dem die oben genannten Kühlmedien bewegt werden.

Die Katalysatoren bestehen aus Trägermaterial und katalytisch wirkender Aktivkomponente. Als Trägermaterial wird üblicherweise aktivierte Tonerde, Aluminiumsilikat, Silicagel oder ähnliches oberflächenaktives Material eingesetzt. Bezüglich der Formgestalt der Katalysatorträger werden zum Beispiel gewäß DE-OS 26 30 938 (=US-A 4,206,180) Formlinge in Kugelgestalt, gemäß DE-OS 31 13 179 (=US-A 4,366,093) Partikel in zylindrischer Form (Hohlzylinder) oder gemäß DE-OS 36 07 449 (=EP-A 240714) Formkörper mit säulenartiger Geometrie verwendet. Formkörper im Zylinder - oder Ringform sind aus der DE-A-3334223 bekannt. Die katalytisch wirkende Aktivkomponente ist weist ein Chlorid und/oder ein Oxid bzw. Oxichlorid von Metallen, bzw. von Kombinationen dieser Metalle mit Promotoren, wie Alkali-, Erdalkali-oder Seltenerdenchloriden. Derartige Metalle wie Kupfer, Mangan, Eisen, Kobalt, Nickel oder Platin weisen die für diese speziellen Anwendungen wirksamen katalytischen Eigenschaften auf.

Daneben verwendet man zur besseren Beherrschung der freiwerdenden Reaktionswärme vielfach Katalysatoren, deren Aktivkomponenten ein in Strömungsrichtung definiert ansteigendes Konzentrationsprofil, bzw. die eine in Strömungsrichtung zunehmende Aktivität haben oder verdünnt zum Teil auch die Aktivkomponente mit inertem Material, wie beispielsweise Graphit (EP-A 60317). Unterschiedliche Aktivitätsstufen eines solchen Katalysators erreicht man nämlich entweder durch Veränderung der Konzentration an Aktivkomponente und/oder durch Variation des molaren Verhältnisses Metallsalz zu Promotor (vgl. DE-OS 26 30 938).

Nachteilig bei Chlorierungs- und Oxichlorierungsprozessen der oben beschriebenen Art ist, daß große Gasmengen (Kohlenwasserstoffe, Chlor und Chlorwasserstoff, Luft und/oder Sauerstoff und Inertgase im Überschuß) durch die Reaktoren geleitet werden, wobei über den einzelnen Reaktoren ein relativ starker Druckabfall entsteht, was den Einsatz von Gebläsen oder Kompressoren notwendig macht, um die gewünschten Gasmengen durch die Reaktoren zu treiben. Der dafür erforderliche Aufwand ist erheblich.

Es bestand daher die Aufgabe durch verfahrenstechnische Maßnahmen den Druckverlust so gering wie möglich zu halten. Weiter bestand die Aufgabe die Ausbildung von punktuellen Überhitzungsstellen, sogenannten Hot-Spots, bei selektiven Chlorierungs- und Oxichlorierungsreaktionen möglichst zu unterdrücken und damit die Ausbeute an Zielprodukt zu erhöhen.

Zur Lösung dieser Aufgabe führt die Verwendung eines monolithischen Katalysatorträgers mit zahlreichen parallel zur Längsachse angeordneten und beidseitig offenen Kanälen und einer, mindestens seinem Durchmesser, vorzugsweise einem Mehrfachen desselben entsprechenden Länge, welcher an seiner, gegebenenfalls mit katalyseförderndem Material überzogen, Oberfläche mit der Katalysatorsubstanz versehen ist, als Katalysator in Rohrbündelreaktoren für selektive Chlorierungs- und/oder Oxichlorierungsreaktionen, insbesonders zur Herstellung von Chlorkohlenwasserstoffen.

Wabenförmige monolithische Katalysatorträger sind bisher überwiegend zur Entgiftung von Autoabgasen eingesetzt worden (DE-A 14 42 653, DE-A 31 00 930). Bei diesem Verfahren herrschen so hohe Reaktionstemperaturen, daß eine Verkokung der Katalysatormatrix nicht zu befürchten ist, da eventuell gebildeter Koks schon im status nascendi mit Sauerstoff abgebrannt wird. Andererseits geraten bei herkömmlichen Chlorierungs- und/oder Oxichlorierungskatalysatoren in Schüttgutform Koksablagerungen sogar in das Korninnere, wodurch das Schüttgut durch Aufplatzen gesprengt wird und damit der Druckabfall im Katalysatorbett dramatisch ansteigen kann. Es war daher zu befürchten, daß bei der erfindungsgemäßen Verwendung monolithischer Katalysatorträger für derartige Chlorierungs- und/oder Oxichlorierungsreaktionen die sehr feinen Kanäle durch Koksablagerung verstopft werden. Überraschenderweise ist dies jedoch nicht der Fall.

Ein in der EP-A 40660 angegebener Einsatz von Wabenkatalysatoren für Oxidations- und Dehydrierungsverfahren ließ eine Eignung für das erfindungsgemäße Arbeitsgebiet mit seiner eigenen Problematik ebensowenig erwarten, wie die Verwendung für Oxidationsreaktionen zur Herstellung von Ethylenoxid, Phtalsäure und Maleinsäureanhydrid (DE-A 32 13 413) oder die in der DE-A 35 21 767 allgemein vorgeschlagene Verwendung für exo- und endotherme chemische Reaktionen mit hoher Wärmetönung unter Einsatz statischer Mischelemente zwischen einzelnen Wabenkörperelementen.

Die monolithischen Katalysatorträger haben eine Länge von einigen Zentimetern bis etwa 20 cm und eine den Reaktorrohren entsprechende Querschnittsform, wobei der innere Durchmesser der Reaktorrohre für derartige stark exotherm verlaufende Reaktionen üblicherweise 20 bis 50 mm beträgt.

Die Querschnittsform ist beliebig, vorzugsweise ist der Querschnitt kreisförmig und der Durchmesser des Trägers geringfügig kleiner als der innere Durchmesser der Reaktionsrohre. Vorzugsweise ist das Durchmesser/Längenverhältnis etwa 1 : 5 bis 1 : 10.

Zur Herstellung solcher Katalysatorträger finden grundsätzlich alle Materialien Anwendung, die bisher auch für herkömmliche Katalysatorformlinge verwendet wurden; zum Beispiel aktivierte Tonerde, Aluminiumsilikat, Silicagel, Titanoxid, Siliciumcarbid oder Mischungen dieser Materialien, Sinterkeramik zum Beispiel aus $\alpha$-Al$_2$O$_3$.

Mellit oder Cordierit wird jedoch bevorzugt. Die Träger werden so behandelt, daß sie im Endzustand poröse Oberflächen aufweisen. Dies kann zum Beispiel durch herstellungstechnische Maßnahmen oder ein- oder mehrmaliges Beschichten eines wenig porösen oder unporösen Trägers mit Tonerde oder SiO$_2$-Hydrat und anschließendes Tempern dieser Überzüge geschehen.

Durch die Verwendung von an sich oberflächenaktiven Materialien, bzw. durch das Beschichten mit oberflächenaktiven Materialien, wie zum Beispiel $\gamma$-Aluminiumoxid, läßt sich die bei der Reaktion für den Übergang Gas-Festzustand erforderliche spezifische Oberfläche einregulieren.

Die Katalysatorträger sind mit parallel zur Längsachse angeordneten, beidseitig offenen Kanälen versehen. Die Geometrie des Querschnitts der Kanäle ist beliebig. Der Durchmesser und die Anzahl der Kanäle, und damit die Größe der äußeren Oberfläche der wabenförmigen Katalysatorträger, sind der betreffenden Reaktion angepaßt. Vorzugsweise beträgt der Durchmesser zwischen 0.1 und 5.0 mm. Der bevorzugte Bereich für die Anzahl der Kanäle liegt bei 10 bis 100 pro cm$^2$ seiner Querschnittsfläche.

Die fertigen Formkörper werden dann entsprechend der erfindungsgemäßen Verwendung mit den üblichen Aktivkomponenten oder Gemischen aus Aktivkomponente und Promotor in den bekannten Konzentrationen imprägniert. Vorzugsweise erfolgt die Imprägnierung des Trägers durch Eintauchen und Tränken in den entsprechenden Lösungen der Aktivkomponente.

Das Füllen der Reaktorrohre geschieht in einfacher Weise dadurch, daß die mit aktiver Katalysatorsubstanz versehenen Katalysatorträger einzeln nacheinander in die Rohre geschoben werden. Bei vorbestimmter Anordnung der Katalysatorträger - und damit auch bei vorbestimmter Anordnung des freien Volumens im Reaktorrohr - läßt sich der Druckabfall über den Reaktor exakt vorausbestimmen und durch den Lochdurchmesser und/oder die Anzahl der Zellen pro Flächeneinheit in jedem einzelnen Katalysatormodul, sowie durch Anbringen einer Abstandshaltung aus vorzugsweise kugelförmigen Füllkörpern zwischen den einzelnen Modulen sehr gering halten. Diese Abstandshaltung wird dabei in der Weise eingestellt, daß man bei der Befüllung abwechselnd Katalysatorformkörper und z.B. Glaskugeln in das Reaktionsrohr einführt. Vorzugsweise beträgt dabei der Durchmesser der Glaskugeln zwischen drei und sechs Millimetern.

Eine bevorzugte Ausführungsform der Erfindung beruht daher in der Verwendung des Katalysatorträgers in hintereinandergeschalteter Mehrfachanordnung des Katalysators innerhalb von Einzelrohren des Rohrbündelreaktors, vorzugsweise unter Zwischenschaltung eines bevorzugt kugelförmigen Füllkörpers zur Abstandshaltung.

Der erfindungsgemäß anzuwendende wabenförmige monolithische Katalysatorträger hat den Vorteil, daß sein spezifisches Gewicht sehr gering ist, und damit zum Befüllen eines vorgegebenen Reaktorvolumens nur wenig Trägermasse erforderlich ist (Kostenverringerung). Durch die zahlreichen Kanäle in Längsrichtung ist, verglichen mit herkömmlichen Schüttgutkatalysatoren, die äußere Oberfläche des Katalysatorträgers pro Volumeneinheit stark erhöht, wodurch die katalytische Aktivität angehoben wird. Weiter wird durch die poröse

3

EP 0 369 439 B1

Oberfläche das Diffusionsvermögen der gasförmigen Reaktanden erhöht. Da die Diffusion der gasförmigen Reaktanden der geschwindigkeitsbestimmende Schritt derartiger Chlorierungs- bzw. Oxichlorierungsreaktionen ist, wird die katalytische Aktivität weiter erhöht. Die Bildung unerwünschter Nebenprodukte, die aus einer unzureichenden Diffusion herrührt, wird verringert. Desgleichen wird der Druckabfall über den einzelnen Reaktor herabgesetzt.

Durch die erfindungsgemäße Verwendung der wabenförmigen monolithischen Katalysatorträger, die nach einer besonders vorteilhaften Ausführungsform der Erfindung mit in Strömungsrichtung zunehmender katalytisch wirkender Aktivität in einen Reaktor gefüllt sind, läßt sich die Reaktion gezielter führen und die Ausbildung von punktuellen Überhitzungsstellen weitgehend unterdrücken.

Die erfindungsgemäße Verwendung erhöht somit die Selektivität von selektiven Chlorierungs- und/oder Oxichlorierungsreaktionen und minimiert die Verbrennungsrate, was letzten Endes zu einer höheren Ausbeute an gewünschtem Produkt führt. Überraschenderweise entfallen auch die gefürchteten Koksablagerungen.

Vorzugsweise werden die erfindungsgemäß zu verwendenden Katalysatorträger für Katalysatoren zur Herstellung von Chlorkohlenwasserstoffen durch selektive Chlorierung- bzw. Oxichlorierung von z.B. Ethylen und/oder Ethan eingesetzt.

Die nachfolgenden Ausführungsbeispiele und Abbildungen dienen zur weiteren Erläuterung der Erfindung.

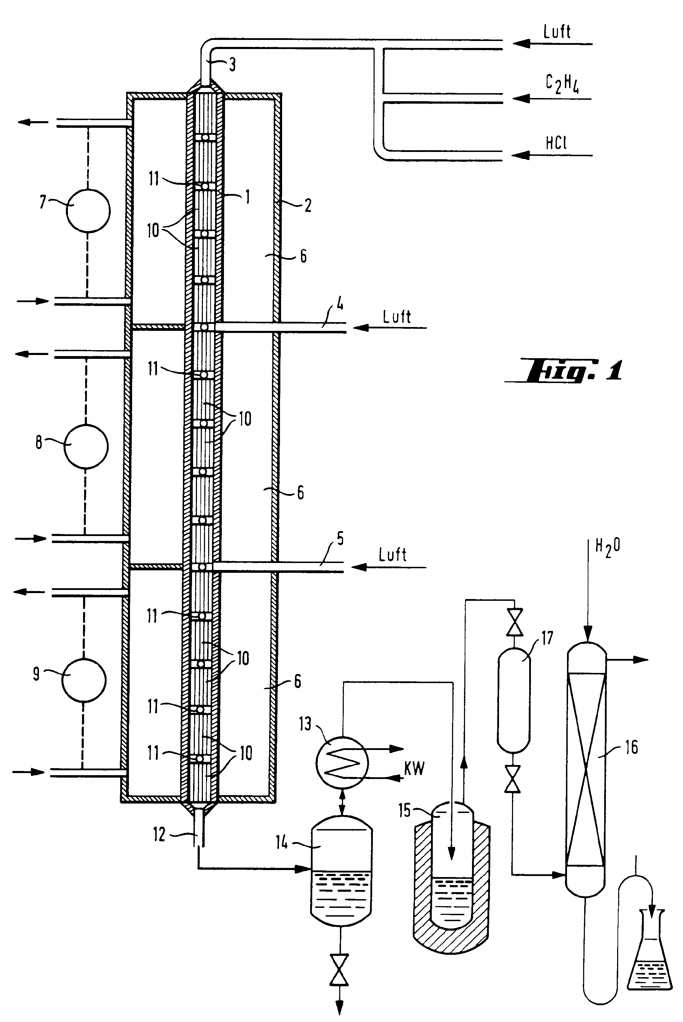

Fig. 1 ist ein schematischer Längsschnitt durch ein mit dem erfindungsgemäßen Katalysatorträger gefülltes Reaktionsrohr wie es in den folgenden Beispielen verwendet wird.

Beispiel 1:

Der Reaktor gemäß Fig. 1 besteht aus einem senkrecht stehendem Nickelrohr 1 mit 25 mm lichter Weite und 2000 mm Länge, das von einem Doppelmantel 2 aus Stahl umschlossen ist. Der Reaktor verfügt über drei Zuleitungen, wobei die Zuleitung 3 am oberen Ende angeordnet ist und zwei weitere Zuleitungen nach dem ersten (Zuleitung 4) bzw. zweiten Drittel (Zuleitung 5) des Reaktionsrohres seitlich angeordnet sind. Im Hohlraum 6 zwischen Nickelrohr 1 und Stahldoppelmantel 2, der vertikal in drei Segmente aufgeteilt ist, wird thermostatisiertes Heizöl umgepumpt. Die Heiz/Kühlkreisläufe jedes Segments sind über die Reglerelemente 7, 8, 9 separat temperaturregelbar. Im oberen Heizkreislauf beträgt die Temperatur 215°C, im mittleren 220°C, im unteren 225°C. Das Reaktionsrohr 1 ist mit Katalysatormodulen 10 derart gefüllt, daß sich zwischen jeweils zwei Modulen mit 115 mm Länge (Ausnahme am Reaktoreintritt bzw. -ausgang, wo keine Glaskugeln benötigt werden) je eine Glaskugel 11 mit 3 mm Durchmesser befindet. Der kreisförmige Durchmesser der Katalysatormodule ist so bemessen, daß die Module gerade in das Reaktionsrohr eingeschoben werden können. Die Trägermodule verfügen über eine zentrisch angebrachte 5 mm-Bohrung, in die, zur Aufnahme des Temperaturprofils, eine mit einem Thermoelement versehene Thermohülse eingeführt wird. Die äußere Oberfläche der keramische Module, die 200 Zellen pro Querschnitt aufwiesen, war durch achtfaches Coaten mit einer 200 $\mu$m-Schicht aus $\gamma$-Al$_2$O$_3$ bedeckt und mit CuCl$_2$ und KCl entsprechend folgendem Befüllplan imprägniert (von oben nach unten):

3 Module mit 6 % CuCl$_2$ und 3 % KCl (35 cm Höhe)
2 Module mit 19 % CuCl$_2$ und 1.8 % KCl (23 cm Höhe)
3 Module mit 10 % CuCl$_2$ und 3 % KCl (35 cm Höhe)
2 Module mit 19 % CuCl$_2$ und 1.8 % KCl (23 cm Höhe)
5 Module mit 19 % CuCl$_2$ und 1.8 % KCl (58 cm Höhe)

Die einzelnen Gasströme werden über geeichte Rotameter zugeführt. 100 Nl/h Chlorwasserstoff und 58 Nl/h Ethylen werden zunächst vermischt und dann zusammen mit 57 Nl/h Luft über Leitung 3 am Oberteil des Reaktors aufgegeben. Weitere 57 Nl/h Luft bzw. 30 Nl/h Luft werden über die Leitungen 4 bzw. 5 zugegeben. Das den Reaktor über Leitung 12 verlassende Produktgemisch wird im Intensivkühler 13 mit Wasser gekühlt, wobei Partialkondensation eintritt. Im Abscheider 14 wird die flüssige Phase abgetrennt. Der Gasstrom in der Kältefalle 15 auf -25°C abgekühlt und im Wasserwäscher 16 HCl-frei gewaschen. Die Kondensate aus dem Abscheider 14 und der Kältefalle 15 werden gesammelt und nach Abtrennen der wässrigen Phase gaschromatographisch analysiert. Das Abgas aus der Kältefalle 15 wird durch Probennahme über die Gasmaus 17 gaschromatographisch auf CO und CO$_2$ untersucht. Der HCl-Umsatz wird aus dem HCl-Gehalt im Ablauf des Wasserwäschers 16 errechnet.

Vergleichsbeispiel 1:

Es wird analog Beispiel 1 verfahren, jedoch anstelle der erfindungsgemäßen Module ein herkömmlicher Trägerkatalysator in Kugelform (Durchmesser 4 - 5 mm; gemäß DE-A 26 30 938) mit $\gamma$-Al$_2$O$_3$ als Trägermaterial

verwendet, wobei das jeweilige Cu/K-Verhältnis dem folgenden Füllplan entspricht (von oben nach unten):

```
35 cm γ-Al₂O₃-Kugeln mit  6 % CuCl₂ und 3   % KCl
23 cm        "       mit 19 % CuCl₂ und 1.8 % KCl
35 cm        "       mit 10 % CuCl₂ und 3   % KCl
23 cm        "       mit 19 % CuCl₂ und 1.8 % KCl
58 cm        "       mit 19 % CuCl₂ und 1.8 % KCl
```

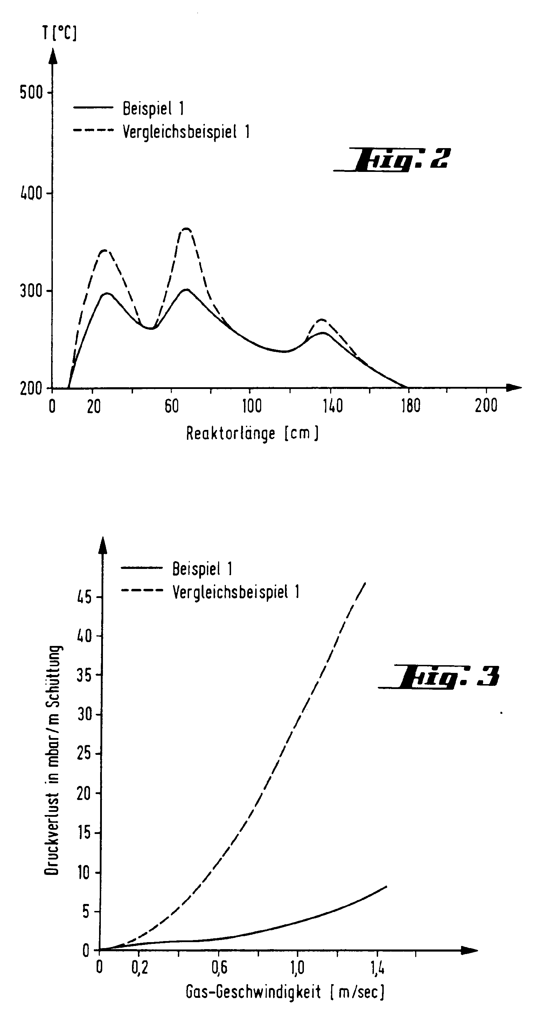

In Fig. 2 bzw. Fig. 3 sind die Temperaturprofile bzw. Druckverluste der Verfahren nach Beispiel 1 und Vergleichsbeispiel 1 gegenübergestellt.

Es wird deutlich, daß die Verwendung der erfindungsgemäßen Katalysatorträger gegenüber einer herkömmlichen Trägerform zu einer erheblich verbesserten Wärmeabführung bei der Oxichlorierung führt (Fig. 2) und die Druckverluste im Reaktor drastisch abgesenkt werden (Fig. 3).

In Tabelle 1 sind die Analysenergebnisse bezüglich Umsatzrate, Selektivität und Verbrennungsrate von Beispiel 1 und Vergleichsbeispiel 1 aufgeführt.

Die Gegenüberstellung in Tabelle 1 zeigt den großen technischen Fortschritt der erzielt wird, wenn man für die genannte Reaktion die erfindungsgemäß vorgesehenen Katalysatormodule einsetzt. Die niedrigere Verbrennungsrate, ausgedrückt als CO- bzw. $CO_2$-Gehalt im Abgas, und die höhere Selektivität bei gleichem HCl-Umsatz, ausgedrückt als Gehalt an 1.2-Dichlorethan im organischen Kondensat, beweisen die Überlegenheit der erfindungsgemäß anzuwendenden Katalysatormodulen gegenüber den bisher für diese Reaktion verwendeten herkömmlichen Trägerkatalysatoren in Kugelform.

Tabelle 1:

|  | Beispiel 1 | Vergleichs-beispiel 1 |
|---|---|---|
| organ. Kondensat | 149.1 $cm^3/h$ | 148.6 $cm^3/h$ |
| wässr. Kondensat | 36.3 $cm^3/h$ | 36.7 $cm^3/h$ |
| Abgasmenge | 145.9 Nl/h | 145.3 Nl/h |
| HCl -Umsatz | 35.2 % | 35.1 % |

Abgasanalyse

|  | Beispiel 1 | Vergleichs-beispiel 1 |
|---|---|---|
| $CO_2$ | 0.78 Vol% | 1.45 Vol% |
| CO | 0.93 Vol% | 1.74 Vol% |

Analyse des
organ. Kondensats

|  | Beispiel 1 | | Vergleichs-beispiel 1 | |
|---|---|---|---|---|
| Ethylchlorid | 0.095 | Gew% | 0.105 | Gew% |
| trans-1.2-Dichlorethylen | 0.033 | " | 0.051 | " |
| 1.1-Dichlorethan | 0.019 | " | 0.013 | " |
| Tetrachlorkohlenstoff | 0.235 | " | 0.337 | " |
| cis-1.2-Dichlorethylen | 0.095 | " | 0.149 | " |
| Chloroform | 0.060 | " | 0.245 | " |
| 1.2-Dichlorethan | 97.43 | " | 95.56 | " |
| Chloral | 0.187 | " | 0.213 | " |
| 1.1.2-Trichlorethan | 1.70 | " | 3.09 | " |

**Patentansprüche**

1.  Verwendung eines wabenförmigen monolithischen Katalysatorträgers mit zahlreichen parallel zur Längsachse angeordneten und beidseitig offenen Kanälen und einer mindestens seinem Durchmesser, vorzugsweise einem Mehrfachen desselben entsprechenden Länge, welcher an seiner, gegebenenfalls mit katalyseförderndem Material überzogenen, Oberfläche mit der Katalysatorsubstanz versehen ist, als Katalysator in Rohrbündelreaktoren für selektive Chlorierungs und/oder Oxichlorierungsreaktionen, insbesonders zur Herstellung von Chlorkohlenwasserstoffen.

2.  Verwendung des Katalysatorträgers nach Anspruch 1, dadurch gekennzeichnet, daß die Anzahl der Kanäle 10 bis 100 pro $cm^2$ seiner Querschnittsfläche beträgt.

**3.** Verwendung des Katalysatorträgers nach Anspruch 1 oder Anspruch 2, dadurch gekennzeichnet, daß das Durchmesser/Längenverhältnis des Katalysatorträgers zwischen 1 : 5 und 1 : 10 beträgt.

**4.** Verwendung des Katalysatorträgers nach den Ansprüchen 1, 2 oder 3 in hintereinandergeschalteter Mehrfachanordnung des Katalysators innerhalb von Einzelrohren des Rohrbündelreaktors, vorzugsweise unter Zwischenschaltung eines bevorzugt kugelförmigen Füllkörpers zur Abstandshaltung.

**5.** Verwendung des Katalysatorträgers nach den Ansprüchen 1, 1, 3 oder 4 in einer Anordnung mehrerer Katalysatormodule im Reaktor mit in Strömungsrichtung zunehmender katalytischer Aktivität.

## Claims

**1.** Use of a monolithic honeycomb catalyst support having a multiplicity of channels, arranged parallel to the longitudinal axis and open at both ends, and a length corresponding at least to its diameter and preferably to a multiple thereof, which support is provided with the catalyst substance on its surface, which may be coated with a catalysis-promoting material, as a catalyst in tube-bundle reactors for selective chlorination reactions and/or oxychlorination reactions, in particular for the production of chlorinated hydrocarbons.

**2.** Use of the catalyst support according to Claim 1, characterised in that the number of channels is 10 to 100 per cm$^2$ of its cross-sectional area.

**3.** Use of the catalyst support according to Claim 1 or 2, characterised in that the diameter/length ratio of the catalyst support is between 1 : 5 and 1 : 10.

**4.** Use of the catalyst support according to Claims 1, 2 or 3, in a series-connected multiple arrangement of the catalyst within individual tubes of the tube-bundle reactor, preferably with the interposition of preferably spherical packing for spacing.

**5.** Use of the catalyst support according to Claims 1, 2, 3 or 4, in an arrangement of a plurality of catalyst modules in a reactor, with the catalytic activity increasing in the direction of flow.

## Revendications

**1.** Utilisation d'un support monolithique de catalyseur, en forme de nids d'abeilles comportant de nombreux canaux ouverts des deux côtés et disposés parallèlement à l'axe longitudinal et ayant une longueur au moins égale à son diamètre et représentant avantageusement un multiple de ce diamètre, support qui comporte à sa surface, éventuellement revêtue d'une matière accélérant la catalyse, une substance à rôle de catalyseur, à titre de catalyseur dans des réacteurs à faisceaux tubulaires pour des réactions sélectives de chloruration et/ou d'oxychloruration, notamment pour la production d'hydrocarbures chlorés.

**2.** Utilisation du support de catalyseur selon la revendication 1, caractérisée en ce que le nombre des canaux est de 10 à 100 par cm$^2$ de sa surface de section transversale .

**3.** Utilisation du support de catalyseur selon la revendication 1 ou la revendication 2, caractérisée en ce que le rapport entre le diamètre et la longueur du support de catalyseur se situe entre 1:5 et 1:10.

**4.** Utilisation du support de catalyseur selon les revendications 1, 2 ou 3, en un agencement multiple, avec disposition l'un derrière l'autre des modules du catalyseur à l'intérieur des tubes individuels du réacteur à faisceaux tubulaires, avantageusement avec intercalation d'un corps de garnissage, de préférence en forme de sphère ou de balle, pour maintenir la distance (entre modules).

**5.** Utilisation de support de catalyseur selon les revendications 1, 2, 3 ou 4, dans un agencement de plusieurs modules dans un réacteur, avec une activité catalytique qui augmente dans le sens de l'écoulement [des corps à faire réagir].

*Fig. 1*

**Fig. 2**

**Fig. 3**